# NEW EUROPEAN PATENT SPECIFICATION

(11) **EP 1 105 529 B2**
(45) Date of publication and mention of the opposition decision: **29.05.2013**
(45) Mention of the grant of the patent: 09.11.2005
(21) Application number: 99936809.5
(22) Date of filing: 30.07.1999
(51) Int. Cl.: C12Q 1/68, B01J 19/00

(54) **ARRAYED BIOMOLECULES AND THEIR USE IN SEQUENCING**
MATRIZEN VON BIOMOLEKÜLEN UND IHRE VERWENDUNG IN SEQUENZIERUNG
BIOMOLECULES EN RANGEES ET LEUR UTILISATION DANS UNE PROCEDURE DE SEQUENCAGE

(30) Priority: 30.07.1998 EP 98306094; 16.10.1998 GB 9822670
(43) Date of publication of application: 13.06.2001
(73) Proprietor: Illumina Cambridge Limited, Little Chesterford Saffron Walden Essex CB10 1XL (GB)
(72) Inventor: BALASUBRAMANIAN, Shankar, Cambridge CB2 1EW (GB); KLENERMAN, David, Cambridge CB2 1EW (GB); BARNES, Colin, Essex CB10 1XL (GB); OSBORNE, Mark Allen, BN1 3JB (GB)
(74) Representative: Baldock, Sharon Claire
(86) International application number: PCT/GB1999/002487
(87) International publication number: WO 2000/006770

(56) References cited:
- EP-A- 0 665 293
- EP-A- 0 853 129
- WO-A-98/20019
- WO-A-98/29736
- DE-A- 19 612 356
- US-A- 5 302 509
- US-A- 5 314 829
- US-A- 5 780 231

### SEQUENCE LISTING

<110> Solexa Ltd
<120> ARRAYED BIOMOLECULES AND THEIR USE IN SEQUENCING
<130> REP05621WO
<140> n/a
   <141> 1999-07-30
<160> 3
<170> PatentIn Ver. 2.1
<210> 1
   <211> 13
   <212> DNA
   <213> Artificial Sequence
<220>
   <223> Description of Artificial Sequence:synthetic
<220>
   <221> misc feature
   <222> (1)..(13)
   <223> Modified base. n = 5'-(propargylamino)uridine
<400> 1
   tcgcagccgn cca 13
<210> 2
   <211> 21
   <212> DNA
   <213> Artificial Sequence
<220>
   <223> Description of Artificial Sequence:synthetic
<220>
   <221> misc_feature
   <222> (1)..(21)
   <223> Modified base. n = 5-methyl cytosine with a TMR group coupled via a linker to the n4 position.
<400> 2
   aaccctatgg acggctgcga n 21
<210> 3
   <211> 21
   <212> DNA
   <213> Artificial Sequence
<220>
   <223> Description of Artificial Sequence:synthetic
<220>
   <221> misc feature
   <222> (1)..(21)
   <223> Modified base. n= methyl cytosine.
<400> 3
   ntcgcagccg tccatagggt t 21

## Description

### Field of the Invention

This invention relates to fabricated arrays of molecules, and to their analytical applications. In particular, this invention relates to the use of fabricated arrays in methods for obtaining genetic sequence information.

### Background of the Invention

Advances in the study of molecules have been led, in part, by improvement in technologies used to characterise the molecules or their biological reactions. In particular, the study of nucleic acids, DNA and RNA, has benefited from developing technologies used for sequence analysis and the study of hybridisation events.

An example of the technologies that have improved the study of nucleic acids, is the development offabricated arrays of immobilised nucleic acids. These arrays typically consist of a high-density matrix of polynucleotides immobilised onto a solid support material. Fodor et al., Trends in Biotechnology (1994) 12:19-26, describes ways of assembling the nucleic acid arrays using a chemically sensitised glass surface protected by a mask, but exposed at defined areas to allow attachment of suitably modified nucleotides. Typically, these arrays may be described as "many molecule" arrays, as distinct regions are formed on the solid support comprising a high density of one specific type of polynucleotide.

An alternative approach is described by Schena et al., Science (1995) 270:467-470, where samples of DNA are positioned at predetermined sites on a glass microscope slide by robotic micropipetting techniques. The DNA is attached to the glass surface along its entire length by non-covalent electrostatic interactions. However, although hybridisation with complementary DNA sequences can occur, this approach may not permit the DNA to be freely available for interacting with other components such as polymerase enzymes, DNA-binding proteins etc.

The arrays are usually provided to study hybridisation events, to determine the sequence ofDNA (Mirzabekov, Trends. in Biotechnology (1994) 12:27-32) or to detect mutations in a particular DNA sample. Many of these hybridisation events are detected using fluorescent labels attached to nucleotides, the labels being detected using a sensitive fluorescent detector, e.g. a charge-coupled detector (CCD). The major disadvantages of these methods are that it is not possible to sequence long stretches of DNA and that repeat sequences can lead to ambiguity in the results. These problems are recognised in Automation Technologies for Genome Characterisation, Wiley-Interscience (1997), ed. T. J. Beugelsdijk, Chapter 10: 205-225.

In addition, the use of high-density arrays in a multi-step analysis procedure can lead to problems with phasing. Phasing problems result from a loss in the synchronisation of a reaction step occurring on different molecules of the array. If some of the arrayed molecules fail to undergo a step in the procedure, subsequent results obtained for these molecules will no longer be instep with results obtained for the other arrayed molecules. The proportion of molecules out of phase will increase through successive steps and consequently the results detected will become ambiguous. This problem is recognised in the sequencing procedure described in US-A-5302509.

An alternative sequencing approach is disclosed in EP-A-0381693, which comprises hybridising a fluorescently-labelled strand of DNA to a target DNA sample suspended in a flowing sample stream, and then using an exonuclease to cleave repeatedly the end base from the hybridised DNA. The cleaved bases are detected in sequential passage through a detector, allowing reconstruction of the base sequence of the DNA. Each of the different nucleotides has a distinct fluorescent label attached, which is detected by laser-induced fluorescence. This is a complex method, primarily because it is difficult to ensure that every nucleotide of the DNA strand is labelled and that this has been achieved with high fidelity to the original sequence.

### Summary of the Invention

The present invention is based in part at least on the realisation that molecule arrays can be produced with sufficient separation between the molecules to provide distinct optical resolution. The arrays may be formed by simply immobilising a mixture of molecules to a solid planar surface in such a way that provides sufficient separation between the molecules to allow each molecule to be resolved optically.

According to the present invention, a device comprises an array of molecules capable of interrogation and immobilised on a solid surface, wherein the array has a surface density which allows each molecule to be individually resolved, e.g. by optical microscopy, and wherein each molecule is immobilised at one or more points, by specific interaction with the surface, other than at that part of each molecule that can be interrogated. Therefore, the arrays ofthe present invention comprise what are effectively single molecules that are more spatially distinct than the arrays of the prior art. This has many important benefits for the study of the molecules and their interaction with other biological molecules. In particular, fluorescence events occurring to each molecule can be detected using an optical microscope linked to a sensitive detector, resulting in a distinct signal for each molecule.

When used in a multi-step analysis of a population of single molecules there is a removal of the phasing problems that are encountered using high density arrays of the prior art. Therefore, the novel arrays also permit a massively parallel approach to monitoring fluorescent or other events on the molecules. Such massively parallel data acquisition makes the arrays extremely useful in a wide range of analysis procedures which involve the screening/characterising of heterogeneous mixtures of molecules. The arrays can be used to characterise a particular synthetic chemical or biological moiety, for example in screening procedures to identify particular molecules produced in combinatorial synthesis reactions.

The arrayed molecules may be immobilised on a solid support via microspheres. A microsphere can be visualised easily, allowing it to be positioned within a distinct optically resolvable region of a microscope prior to carrying out further analysis procedures.

The arrays may be used in many different analysis procedures or characterisation studies. In one embodiment, the molecules are polynucleotides, and the arrays permit sequence determinations to be carried out.

Generally, any sequencing method can be used which makes use of fluorescent or other labels to identify particular nucleotides or sequences of nucleotides. A preferred method comprises the repeated steps of: reacting an immobilised target polynucleotide with a primer, a polymerase and the different nucleoside triphosphates under conditions sufficient for the polymerase reaction to proceed, wherein each nucleoside triphosphate is conjugated at its 3' position to a different fluorescent label, determining which label (and thus which nucleotide) has undergone the polymerase reaction, and removing the label. Because the method utilises the arrays of the present invention, each incorporated nucleotide can be unambiguously determined by fluorescent measurements, and additionally the method can be used to detect many thousands of reactions at the same time with no phasing problems.

Alternatively, the arrays may be used in genotyping procedures (as disclosed in Shalon et al, Genome Research (1996) 639-645), to provide a genetic "bar code" for an organism, mapping studies and mRNA-based expression monitoring (as disclosed in Wodicka et al, Nat. Biotechnol. (1997) 15:1359). The arrays may also be used as a sensor, in the manner disclosed in Analytical Chemistry (1998) 70:1242-1248.

According to a further aspect of the invention, a method comprises contacting, under suitable conditions, an immobilised array of polynucleotides according to the present invention, of predetermined sequence, with a plurality of target molecules capable of binding to the arrayed polynucleotides, and detecting a binding event, thereby determining the position of a bound molecule on the array. This method permits identification of molecules synthesised by the combinatorial chemistry reactions and incorporating, for example, a polynucleotide identifier tag.

A further method comprises the steps of contacting an array of polynucleotides according to the invention with a plurality of detectably-labelled fragments of an organism's genomic DNA, under hybridising conditions, and detecting hybridisation events. The organism may be mammalian, in particular human, or alternatively the organism may be bacterial or viral. This method allows genotyping analysis to be carried out.

An array of the invention may be used to generate a spatially addressable array of single polynucleotide molecules. This is the simple consequence of sequencing the array. Particular advantages of such a spatially addressable array include the following:
1) Polynucleotide molecules on the array may act as identifier tags and may only need to be 10-20 bases long, and the efficiency required in the sequencing steps may only need to be better than 95%.
2) The arrays may be reusable for screening once created and sequenced. All possible sequences can be produced in a very simple way, e.g. compared to a high density DNA chip made using photolithography.

### Description of the Drawings

Figure 1 is a schematic representation of apparatus that may be used to image arrays of the present invention;
Figure 2 illustrates the immobilisation of a polynucleotide to a solid surface via a microsphere;
Figure 3 shows a fluorescence time profile from a single fluorophore-labelled oligonucleotide, with excitation at 514nm and detection at 600nm;
Figure 4 shows fluorescently labelled single molecule DNA covalently attached to a solid surface; and
Figure 5 shows images of surface bound oligonucleotides hybridised with the complementary sequence.

### Description of the Invention

According to the present invention, the single molecules immobilised onto the surface of a solid support must be capable of being individually resolved, e.g. by optical means. This means that, within the resolvable area of the particular imaging device used, there must be one or more distinct images each representing one molecule. Typically, the molecules of the array are resolved using a single molecule fluorescence microscope equipped with a sensitive detector, e.g. a charge-coupled detector (CCD), each molecule of the array being analysed simultaneously.

The molecules of the array may be any biomolecule including peptides and polypeptides, but in particular DNA and RNA and nucleic acid mimics, e.g. PNA and 2'-O-methRNA. However, other organic molecules may also be used. The molecules are formed on the array to allow interaction with other "cognate" molecules. It is therefore important to immobilise the molecules so that the portion of the molecule not used to immobilise the molecule, is capable of being interrogated by a cognate. In some applications all the molecules in the single array will be the same, and may be used to interrogate molecules that are largely distinct. In other applications, the molecules on the array will primarily be distinct, e.g. more than 50%, preferably more than 70% of the molecules will be different to that of the other molecules.

The arrays of the present invention are single molecule arrays. The term "single molecule" is used herein to refer to one molecule that is visualised separately from neighbouring molecules (whether or not each molecule is of the same or different type).

The term "individually resolved" is used herein to specify that, when visualised, it is possible to distinguish one molecule on the array from its neighbouring molecules. Visualisation is effected by the use of reporter labels, e.g. fluorophores, the signal of which is individually resolved.

The term "cognate molecule" is used herein to refer to any molecule capable of interacting, or interrogating, the arrayed molecule. The cognate may be a molecule that binds specifically to the arrayed molecule, for example a complementary polynucleotide, in a hybridisation reaction. Alternatively, the cognate may associate non-specifically with the arrayed molecule, for example a polymerase enzyme which associates with an arrayed polynucleotide in the process of synthesising a complementary strand.

The term "interrogate" is used herein to refer to any interaction of the arrayed molecule with any other molecule. The interaction may be covalent or non-covalent.

The terms "arrayed polynucleotides" and "polynucleotide arrays" are used herein to define an array of single molecules that are characterised by comprising a polynucleotide molecule. The term is intended to include the attachment of other molecules to a solid surface, the molecules having a polynucleotide attached that can be further interrogated. For example, the arrays may comprise protein molecules immobilised on a solid surface, the protein molecules being conjugated with or otherwise bound to a short polynucleotide molecule may be interrogated, to address the array.

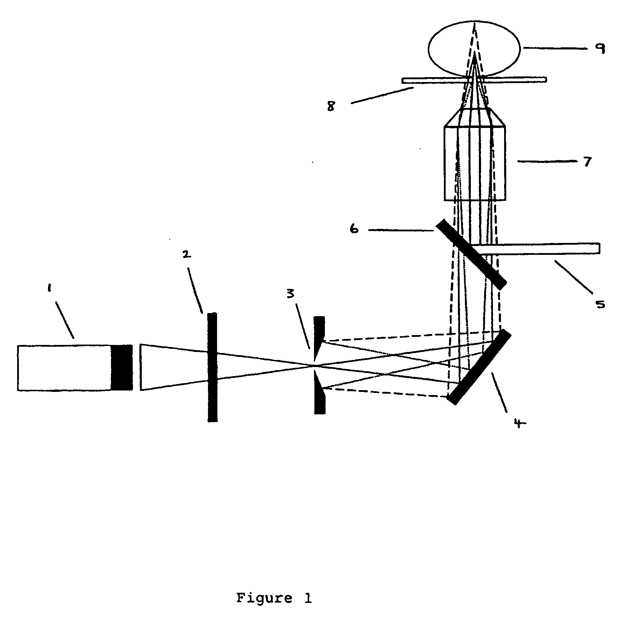

The extent of separation between the individual molecules on the array will be determined, in part, by the particular technique used to resolve the individual molecule. Apparatus used to image molecular arrays are known to those skilled in the art. For example, a confocal scanning microscope may be used to scan the surface of the array with a laser to image directly a fluorophore incorporated on the individual molecule by fluorescence, as shown in Figure 1, where (1) represents a detector, (2) a bandpass filter, (3) a pinhole, (4) a mirror, (5) a laser beam, (6) a dichroic mirror, (7) an objective, (8) a glass coverslip and (9) a sample under study. Alternatively, a sensitive 2-D detector, such as a charge-coupled detector, can be used to provide a 2-D image representing the individual molecules on the array. In this example, resolving single molecules on the array is possible if the molecules are separated by a distance of approximately at least 250nm x 250nm, preferably at least 300nm x 300nm and more preferably by at least 350nm x 350nm.

However, other techniques such as scanning near-field optical microscopy (SNOM) are available which are capable of smaller optical resolutions, thereby permitting "more dense" arrays to be used. For example, using SNOM, the molecules may be separated by a distance of less than 100nm, e.g. 10nm x 10nm. For a description of scanning near-field optical microscopy, see Moyer et al., Laser Focus World (1993) 29(10).

Additionally, the techniques of scanning tunnelling microscopy (Binnig et al., Helvetica Physica Acta (1982) 55:726-735) and atomic force microscopy (Hanswa et al., Annu. Rev. Biophys. Biomol. Struct. (1994) 23:115-139) are suitable for imaging the arrays of the present invention. Other devices which do not rely on microscopy may also be used, provided that they are capable of imaging within discrete areas on a solid support.

Single molecules may be arrayed by immobilisation to the surface of a solid support. This may be carried out by any known technique, provided that suitable conditions are used to ensure adequate separation of the molecules. Generally the array is produced by dispensing small volumes of a sample containing a mixture of molecules onto a suitably prepared solid surface, or by applying a dilute solution to the solid surface to generate a random array. In this manner, a mixture of different molecules may be arrayed by simple means. The formation of the single molecule array then permits identification of each arrayed molecule to be carried out.

It is important to prepare the solid support under conditions which minimise or avoid the presence of contaminants. The solid support must be cleaned thoroughly, preferably with a suitable detergent, e.g. Decon-90, to remove dust and other contaminants.

Immobilisation may be by specific covalent or non-covalent interactions. If the molecule is a polynucleotide, immobilisation will preferably be at either the 5' or 3' position, so that the polynucleotide is attached to the solid support at one end only. However, the polynucleotide may be attached to the solid support at any position along its length, the attachment acting to tether the polynucleotide to the solid support. The immobilised polynucleotide is then able to undergo interactions with other molecules or cognates at positions distant from the solid support. Typically the interaction will be such that it is possible to remove any molecules bound to the solid support through nonspecific interactions, e.g. by washing. Immobilisation in this manner results in well separated single molecules. The advantage of this is that it prevents interaction between neighbouring molecules on the array, which may hinder interrogation of the array

In one embodiment of the invention, the surface of a solid support is first coated with streptavidin or avidin, and then a dilute solution of a biotinylated molecule is added at discrete sites on the surface using, for example, a nanolitre dispenser to deliver one molecule on average to each site. If the molecule is a polynucleotide, then immobilisation may be via hybridisation to a complementary nucleic acid molecule previously attached to a solid support. For example, the surface of a solid support may be first coated with a primer polynucleotide at discrete sites on the surface. Single-stranded polynucleotides are then brought into contact with the arrayed primers under hybridising conditions and allowed to "self-sort" onto the array. In this way, the arrays may be used to separate the desired polynucleotides from a heterogeneous sample of polynucleotides.

Alternatively, the arrayed primers may be composed of double-stranded polynucleotides with a single-stranded overhang ("sticky-ends"). Hybridisation with target polynucleotides is then allowed to occur and a DNA ligase used to covalently link the target DNA to the primer. The second DNA strand can then be removed under melting conditions to leave an arrayed polynucleotide.

In a preferred embodiment of the invention, the solid surface is coated with an epoxide and the molecules are coupled via an amine linkage. It is also preferable to avoid or reduce salt present in the solution containing the molecule to be arrayed. Reducing the salt concentration minimises the possibility of the molecules aggregating in the solution, which may affect the positioning on the array.

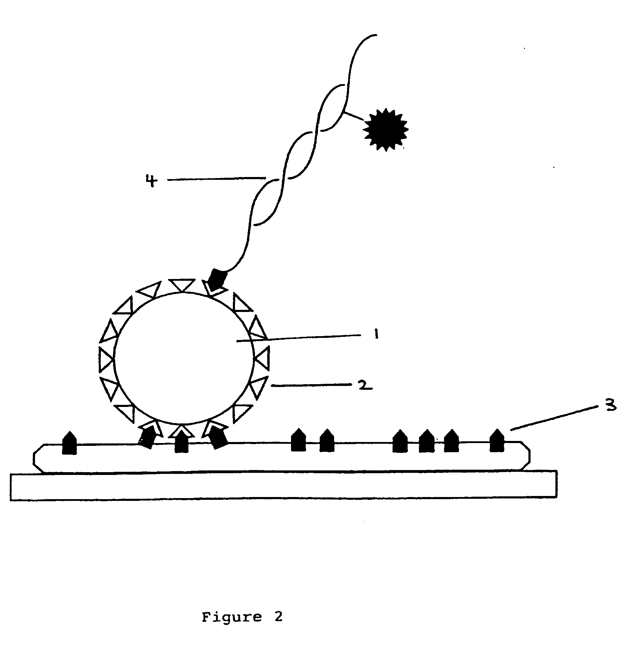

In an embodiment of the invention, the target molecules are immobilised onto non-fluorescent streptavidin or avidin-functionalised polystyrene latex microspheres, as shown in Fig. 2 where (1) represents the microsphere, (2) a streptavidin molecule (3) a biotin molecule and (4) a fluorescently labelled polynucleotide. The microspheres are immobilised in turn onto a solid support to fix the target sample for microscope analysis. Alternative microspheres suitable for use in the present invention are well known in the art.

The single molecule arrays have many applications in methods which rely on the detection ofbiological or chemical interactions with arrayed molecules. For example, the arrays may be used to determine the properties or identities of cognate molecules. Typically, interaction of biological or chemical molecules with the arrays are carried out in solution.

In particular, the arrays may be used in conventional assays which rely on the detection of fluorescent labels to obtain information on the arrayed molecules. The arrays are particularly suitable for use in multi-step assays where the loss of synchronisation in the steps was previously regarded as a limitation to the use of arrays. When the arrays are composed of polynucleotides they may be used in conventional techniques for obtaining genetic sequence information. Many of these techniques rely on the stepwise identification of suitably labelled nucleotides, referred to in US-A-5634413 as "single base" sequencing methods.

In an embodiment of the invention, the sequence of a target polynucleotide is determined in a similar manner to that described in US-A-5634413, by detecting the incorporation of nucleotides into the nascent strand through the detection of a fluorescent label attached to the incorporated nucleotide. The target polynucleotide is primed with a suitable primer, and the nascent chain is extended in a stepwise manner by the polymerase reaction. Each of the different nucleotides (A, T, G and C) incorporates a unique fluorophore at the 3' position which acts as a blocking group to prevent uncontrolled polymerisation. The polymerase enzyme incorporates a nucleotide into the nascent chain complementary to the target, and the blocking group prevents further incorporation of nucleotides. The array surface is then cleared of unincorporated nucleotides and each incorporated nucleotide is "read" optically by a charge-coupled detector using laser excitation and filters. The 3' -blocking group is then removed (deprotected), to expose the nascent chain for further nucleotide incorporation.

Because the array consists of distinct optically resolvable polynucleotides, each target polynucleotide will generate a series of distinct signals as the fluorescent events are detected. Details of the full sequence are then determined.

The number of cycles that can be achieved is governed principally by the yield of the deprotection cycle. If deprotection fails in one cycle, it is possible that later deprotection and continued incorporation of nucleotides can be detected during the next cycle. Because the sequencing is performed at the single molecule level, the sequencing can be carried out on different polynucleotide sequences at one time without the necessity for separation of the different sample fragments prior to sequencing. This sequencing also avoids the phasing problems associated with prior art methods.

Deprotection may be carried out by chemical, photochemical or enzymatic reactions.

A similar, and equally applicable, sequencing method is disclosed in EP-A-0640146.

Other suitable sequencing procedures will be apparent to the skilled person. In particular, the sequencing method may rely on the degradation of the arrayed polynucleotides, the degradation products being characterised to determine the sequence.

An example ofa suitable degradation technique is disclosed in WO-A- 95/20053, whereby bases on a polynucleotide are removed sequentially, a predetermined number at a time, through the use of labelled adaptors specific for the bases, and a defined exonuclease cleavage.

However, a consequence of sequencing using non-destructive methods is that it is possible to form a spatially addressable array for further characterisation studies, and therefore non-destructive sequencing may be preferred. In this context, term "spatially addressable" is used herein to describe how different molecules may be identified on the basis of their position on an array.

Once sequenced, the spatially addressed arrays may be used in a variety of procedures which require the characterisation of individual molecules from heterogeneous populations.

One application is to use the arrays to characterise products synthesised in combinatorial chemistry reactions. During combinatorial synthesis reactions, it is usual for a tag or label to be incorporated onto a beaded support or reaction product for the subsequent characterisation of the product. This is adapted in the present invention by using polynucleotide molecules as the tags, each polynucleotide being specific for a particular product, and using the tags to hybridise onto a spatially addressed array. Because the sequence of each arrayed polynucleotide has been determined previously, the detection of an hybridisation event on the array reveals the sequence of the complementary tag on the product. Having identified the tag, it is then possible to confirm which product this relates to. The complete process is therefore quick and simple, and the arrays may be reused for high through-put screening. Detection may be carried out by attaching a suitable label to the product, e.g. a fluorophore.

Combinatorial chemistry reactions may be used to synthesise a diverse range of different molecules, each of which may be identified using the addressed arrays of the present invention. For example, combinatorial chemistry may be used to produce therapeutic proteins or peptides that can be bound to the arrays to produce an addressed array of target proteins. The targets may then be screened for activity, and those proteins exhibiting activity may be identified by their position on the array as outlined above.

Similar principles apply to other products of combinatorial chemistry, for example the synthesis of non-polymeric molecules of M.wt.<1000. Methods for generating peptides/proteins by combinatorial methods are disclosed in US-A-5643768 and US-A-5658754. Split-and-mix approaches may also be used, as described in Nielsen et al., J. Am. Chem. Soc. (1993) 115:9812-9813.

In an alternative approach, the products of the combinatorial chemistry reactions may comprise a second polynucleotide tag not involved in the hybridisation to the array. After formation by hybridisation, the array may be subjected to repeated polynucleotide sequencing to identify the second tag which remains free. The sequencing may be carried out as described previously.

Therefore, in this application, it is the tag that provides the spatial address on the array. The tag may then be removed from the product by, for example, a cleavable linker, to leave an untagged spatially addressed array.

A further application is to display proteins via an immobilised polysome containing trapped polynucleotides and protein in a complex, as described in US 5643768 and US 5658754.

In a separate embodiment of the invention, the arrays may be used to characterise an organism. For example, an organism's genomic DNA may be screened using the arrays, to reveal discrete hybridisation patterns that are unique to an individual. This embodiment may therefore be likened to a "bar code" for each organism. The organism's genomic DNA may be first fragmented and detectably-labelled, for example with a fluorophore. The fragmented DNA is then applied to the array under hybridising conditions and any hybridisation events monitored.

Alternatively, hybridisation may be detected using an in-built fluorescence based detection system in the arrayed molecule, for example using the "molecular beacons" described in Nature Biotechnology (1996) 14:303-308.

It is possible to design the arrays so that the hybridisation pattern generated is unique to the organism and so could be used to provide valuable information on the genetic character of an individual. This may have many useful applications in forensic science. Alternatively, the methods may be carried out for the detection of mutations or allelic variants within the genomic DNA of an organism.

For genotyping, it is desirable to identify if a particular sequence is present in the genome. The smallest possible unique oligomer is a 16-mer (assuming randomness of the genome sequence), i.e. statistically there is a probability of any given 16-base sequence occurring only once in the human genome (which has 3 x 10⁹ bases). There are c.4 x 10⁹ possible 16-mers which would fit within a region of 2 cm x 2 cm (assuming a single copy at a density of 1 molecule per 250 nm x 250 nm square). It is therefore necessary to determine only if a particular 16-mer is present or not, and so quantitative measurements are unnecessary. Identifying a mutation in a particular region and what the mutation is can be carried out using the 16-mer library. Mapping back onto the human genome would be possible using published data and would not be a problem once the entire genome has been determined. There is built-in self-check, by looking at the hybridisation to particular 16-mers so that if there is a single point mutation, this will show up in 16 different 16-mers, identifying a region of 32 bases in the genome (the mutation would occur at the top of one 16-mer and then at the second base in a related 16-mer etc). Thus, a single point mutation would result in 16 of the 16- mers not showing hybridisation and a new set of 16 showing hybridisation plus the same thing for the complementary strand. In summary, considering both strands of DNA, a single point mutation would result in 32 of the 16-mers not showing hybridisation and 32 new 16-mers showing hybridisation, i.e. quite large changes on the hybridisation pattern to the array.

By way ofexample, a sample of human genomic DNA may be restriction-digested to generate short fragments, then labelled using a fluorescently-labelled monomer and a DNA polymerase or a terminal transferase enzyme. This produces short lengths of sample DNA with a fluorophore at one end. The melted fragments may then be exposed to the array and the pixels where hybridisation occurs or not would be identified. This produces a genetic bar code for the individual with (if oligonucleotides of length 16 were used) c.4 x 10⁹ binary coding elements. This would uniquely define a person's genotype for pharmagenomic applications. Since the arrays should be reusable, the same process could be repeated on a different individual.

Viral and bacterial organisms may also be studied, and screening nucleic acid samples may reveal pathogens present in a disease, or identify microorganisms in analytical techniques. For example, pathogenic or other bacteria may be identified using a series of single molecule DNA chips produced from different strains of bacteria. Again, these chips are simple to make and reusable.

In a further example, double-stranded arrays may be used to screen protein libraries for binding, using fluorescently labelled proteins. This may determine proteins that bind to a particular DNA sequence, i.e. proteins that control transcription. Once the short sequence that the protein binds to has been determined, it may be made and affinity purification used to isolate and identify the protein. Such a method could find all the transcription-controlling proteins. One such method is disclosed in Nature Biotechnology (1999) 17:p573-577.

Another use is in expression monitoring. For this, a label is required for each gene. There are c. 100,000 genes in the human genome. There are 262,144 possible 9-mers, so this is the minimum length of oligomer needed to have a unique tag for each gene. This 9-mer label needs to be at a specific point in the DNA and the best point is probably immediately after the poly-A tail in the mRNA (i.e. a 9-mer linked to a poly-T guide sequence). Multiple copies of these 9-mers should be present, to permit quantitation of gene expression. 100 copies would allow determination of relative expression from 1-100%. 10,000 copies would allow determination of relative gene expression from .01-100%. 10,000 copies of 262,144 9-mers would fit inside 1 cm x 1 cm at close to maximum density.

The use of nanovials in conjunction with any of the above methods may allow a molecule to be cleaved from the surface, yet retain its spatial integrity. This permits the generation of spatially addressable arrays of single molecules in free solution, which may have advantages where the surface attachment impedes the analysis (e.g. drug screening). A nanovial is a small cavity in a flat glass surface, e.g. approx 20 µm in diameter and 10 µm deep. They can be placed every 50 µm, and so the array would be less dense than a surface-attached array; however, this could be compensated for by appropriate adjustment in the imaging optics.

The following Examples illustrate the invention, with reference to the accompanying drawings.

### Example 1

The microscope set-up used in the following Example was based on a modified confocal fluorescence system using a photon detector as shown in Figure 1. Briefly, a narrow, spatially filtered laser beam (CW Argon Ion Laser Technology RPC50) was passed through an acousto-optic modulator (AOM) (A.A Opto-Electronic) which acts as a fast optical switch. The acousto-optic modulator was switched on and the laser beam was directed through an oil emersion objective (100 X, NA = 1.3) of an inverted optical microscope (Nikon Diaphot 200) by a dichroic beam splitter (540DRLP02 or 505DRLP02, Omega Optics Inc.). The objective focuses the light to a diffraction-limited spot on the target sample immobilised on a thin glass coverslip. Fluorescence from the sample was collected by the same objective, passed through the dichroic beam splitter and directed through a 50 µm pinhole (Newport Corp.) placed in the image plane ofthe microscope observation port. The pinhole rejects light emerging from the sample which is out of the plane of the laser focus. The transmitted fluorescence was separated spectrally by a dichroic beam splitter into red and green components which was filtered to remove residual laser scatter. The remaining fluorescence components were then focused onto separate single photon avalanche diode detectors and the signals recorded onto a multichannel scalar (MCS) (MCS-Plus, EG & G Ortec) with time resolutions in the 1 to 10 ms range.

The target sample was a 5'-biotin-modified 13-mer primer oligonucleotide prepared using conventional phosphoramidite chemistry, and having SEQ ID No. 1 (see listing, below). The oligonucleotide was post-synthetically modified by reaction of the uridine base with the succinimdyl ester of tetramethylrhodamine (TMR).

Glass coverslips were prepared by cleaning with acetone and drying under nitrogen. A 50 µl aliquot ofbiotin-BSA (Sigma) redissolved in PBS buffer (0.01 M, pH 7.4) at 1 mg/ml concentration was deposited on the clean coverslip and incubated for 8 hours at 30°C. Excess biotin-BSA was removed by washing 5 times with MilliQ water and drying under nitrogen. Non-fluorescent streptavidin functionalised polystyrene latex microspheres of diameter 500nm (Polysciences Inc.) were diluted in 100 mM NaCl to 0.1 solids and deposited as a 1 µl drop on the biotinylated coverslip surface. The spheres were allowed to dry for one hour and unbound beads removed by washing 5 times with MilliQ water. This procedure resulted in a surface coverage of approximately 1 sphere/100 µm x 100 µm.

The non-fluorescent microspheres were found to have a broad residual fluorescence at excitation wavelength 514nm, probably arising from small quantities of photoactive constituents used in the colloidal preparation of the microspheres. The microspheres were therefore photobleached by treating the prepared coverslip in a laser beam of a frequency doubled (532nm) Nd:YAG pulsed dye laser, for 1 hour.

The biotinylated 13-TMR ssDNA was coupled to the streptavidin functionalised microspheres by incubating a 50 µl sample of 0.1 pM DNA (diluted in 100 mM NaCl, 100 mM Tris) deposited over the microspheres. Unbound DNA was removed by washing the coverslip surface 5 times with MilliQ water.

Low light level illumination from the microscope condenser was used to position visually a microsphere at 10x magnification so that when the laser was switched on the sphere was located in the centre of the diffraction limited focus. The condenser was then turned off and the light path switched to the fluorescence detection port. The MCS was initiated and the fluorescence omitted from the latex sphere recorded on one or both channels. The sample was excited at 514nm and detection was made on the 600nm channel.

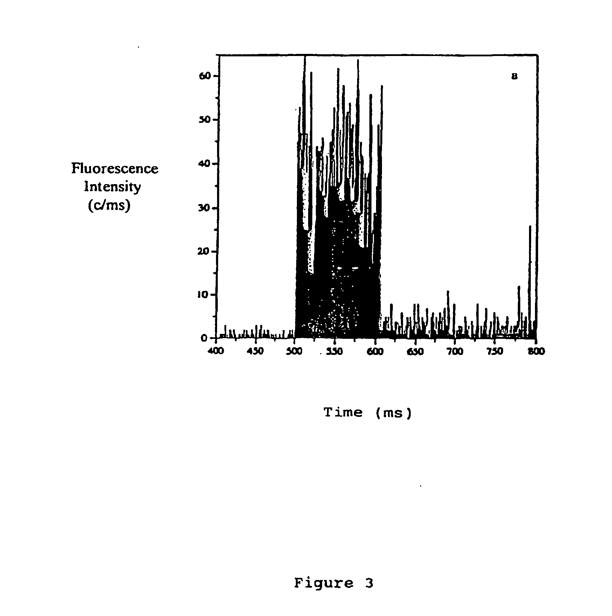

Figure 3 shows clearly that the fluorescence is switched on as the laser is deflected into the microscope by the AOM, 0.5 seconds after the start of a scan. The intensity of the fluorescence remains relatively constant for a short period of time (100 ms-3s) and disappears in a single step process. The results show that single molecule detection is occurring. This single step photobleaching is unambiguous evidence that the fluorescence is from a single molecule.

### Example 2

This Example illustrates the preparation of single molecule arrays by direct covalent attachment to glass followed by a demonstration of hybridisation to the array.

Covalently modified slides were prepared as follows. Spectrosil-2000 slides (TSL, UK) were rinsed in milli-Q to remove any dust and placed wet in a bottle containing neat Decon-90 and left for 12 h at room temperature. The slides were rinsed with milli-Q and placed in a bottle containing a solution of 1.5% glycidoxypropyltrimethoxy-silane in milli-Q and magnetically stirred for 4 h at room temperature rinsed with milli-Q and dried under N₂ to liberate an epoxide coated surface.

The DNA used was that shown in SEQ ID No. 2 (see sequence listing below), where n represents a 5-methyl cytosine (Cy5) with a TMR group coupled via a linker to the n4 position.

A sample of this (5 µl, 450 pM) was applied as a solution in neat milli-Q.

The DNA reaction was left for 12 h at room temperature in a humid atmosphere to couple to the epoxide surface. The slide was then rinsed with milli-Q and dried under N₂.

The prepared slides can be stored wrapped in foil in a desiccator for at least a week without any noticeable contamination or loss of bound material. Control DNA of the same sequences and fluorophore but without the 5'-amino group shows little stable coverage when applied at the same concentration.

The TMR labelled slides were then treated with a solution of complementary DNA (SEQ ID No. 3) (5µM, 10µl) in 100mM PBS. The complementary DNA has the sequence shown in SEQ ID No. 3, where n represents a methylcytosine group.

After 1 hour at room temperature the slides were cooled to 4°C and left for 24 hours. Finally, the slides were washed in PB S (100mM, 1mL) and dried under N₂.

A chamber was constructed on the slide by sealing a coverslip (No. 0, 22x22mm, Chance Propper Ltd, UK) over the sample area on two sides only with prehardened microscope mounting medium (Eukitt, O. Kindler GmbH & Co., Freiburg, Germany) whilst maintaining a gap of less than 200µm between slide and coverslip. The chamber was flushed 3x with 100µl PBS (100mM NaCl) and allowed to stabilise for 5 minutes before analysing on a fluorescence microscope.

The slide was inverted so that the chamber coverslip contacted the objective lens of an inverted microscope (Nikon TE200) via an immersion oil interface. A 60° fused silica dispersion prism was optically coupled to the back of the slide through a thin film of glycerol. Laser light was directed at the prism such that at the glass/sample interface it subtends an angle of approximately 68° to the normal of the slide and subsequently undergoes Total Internal Reflection (TIR). The critical angle for glass/water interface is 66°.

Flurorescence from single molecules of DNA-TMR or DNA-Cy5 produced by excitation with the surface specific evanescent wave following TIR is collected by the objective lens of the microscope and imaged onto an Intensified Charge Coupled Device (ICCD) camera (Pentamax, Princeton Instruments, NJ). Two images were recorded using a combination of 1) 532nm excitation (frequency doubled solid state Nd:YAG, Antares, Coherent) with a 580nm fluorescence (580DF30, Omega Optics, USA) filter for TMR and 2) 630nm excitation (nd:YAG pumped dye laser, Coherent 700) with a 670nm filter (670DF40, Omega Optics, USA) for Cy5. Images were recorded with an exposure time of 500ms at the maximum gain of 10 on the ICCD. Laser powers incident at the prism were 50mW and 40mW at 532nm and 630nm respectively. A third image was taken with 532nm excitation and detection at 670nm to determine the level of crosstalk from TMR on the Cy5 channel.

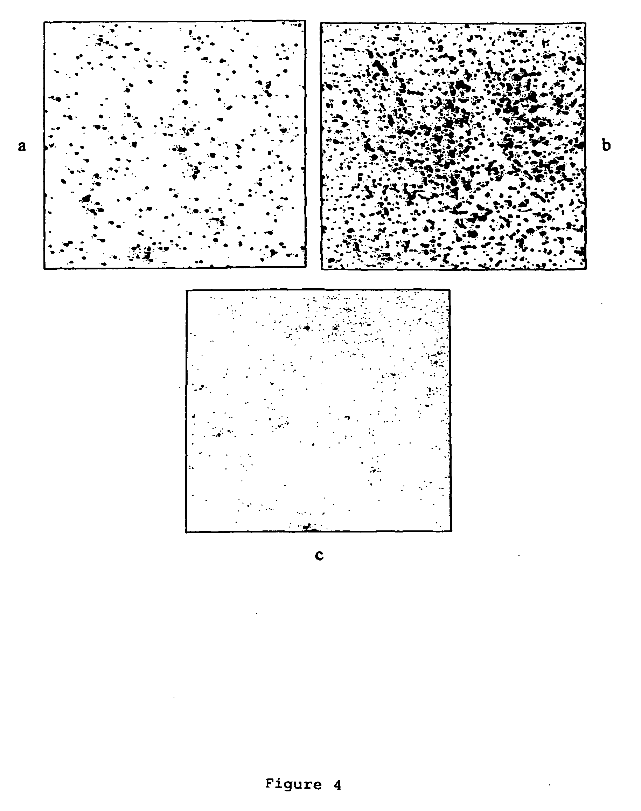

Single molecules were identified by single points of fluorescence with average intensities greater than 3x that of the background. Fluorescence from a single molecule is confined to a few pixels, typically a 3x3 matrix at 100x magnification, and has a narrow Gaussian-like intensity profile. Single molecule fluorescence is also characterised by a one-step photobleaching process in the time course of the intensity and was used to distinguish single molecules from pixel regions containing two or more molecules, which exhibited multi-step processes. Figures 4a and 4b show 60x60µm² fluorescence images from covalently modified slides with DNA-TMR starting concentrations of 45pM and 450pm. Figure 4c shows a control slide which was treated as above but with DNA-TMR lacking the 5' amino modification.

To count molecules a threshold for fluorescence intensities is first set to exclude background noise. For a control sample the background is essentially the thermal noise of the ICCD measured to be 76 counts with a standard deviation of only 6 counts. A threshold is arbitrarily chosen as a linear combination of the background, the average counts over an image and the standard deviation over an image. In general, the latter two quantities provide a measure of the number of pixels and range of intensities above background. This method gives rise to threshold levels which are at least 12 standard deviations above the background with a probability of less than 1 in 144 pixels contributing from noise. By defining a single molecule fluorescent point as being at least a 2x2 matrix of pixels and no larger than a 7x7, the probability of a single background pixel contributing to the counting is eliminated and clusters are ignored.

In this manner, the surface density of single molecules of DNA-TMR is measured at 2.9x10⁶ molecules/cm² (238 molecules in Figure 4a) and 5.8x10⁶ molecules/cm² (469 molecules in Figure 4b) at 45pM and 450pM DNA-TMR coupling concentrations. The density is clearly not directly proportional to DNA concentration but will be some function of the concentration, the volume of sample applied, the area covered by the sample and the incubation time. The percentage of non-specifically bound DNA-TMR and impurities contribute of the order of 3-9% per image (8 non-specifically bound molecules in Figure 4c). Analysis of the photobleaching profiles shows only 6% of fluorescence points contain more than 1 molecule.

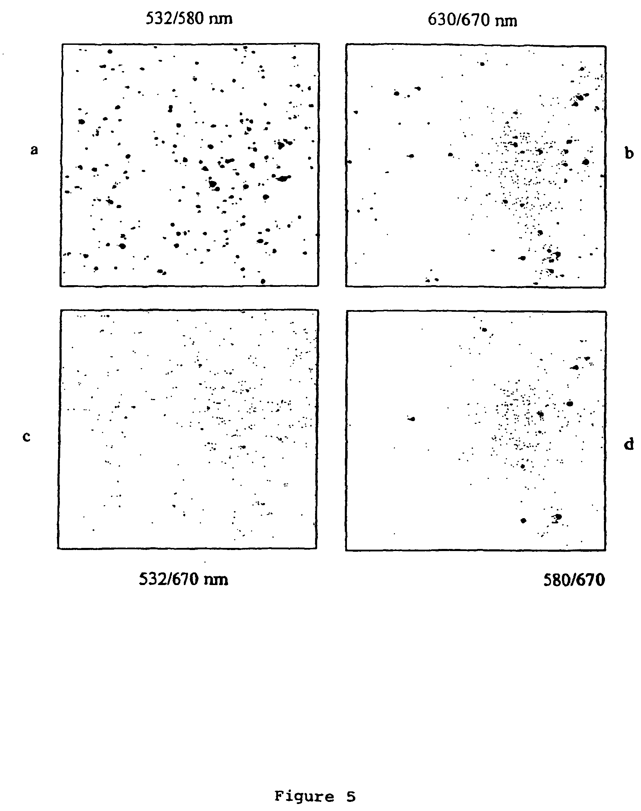

Hybridisation was identified by the co-localisation of discreet points of fluorescence from single molecules of TMR and Cy-5 following the superposition oftwo images. Figures 5a and 5b show images of surface bound 20-mer labelled with TMR and the complementary 20-mer labelled with Cy-5 deposited from solution. Figure 5d shows those fluorescent points that are co-localised on the two former images. The degree of hybridisation was estimated to be 7% ofthe surface- bound DNA (10 co-localised points in 141 points from Figures 5d and 5a respectively). The percentage of hybridised DNA is estimated to be 37% of all surface-adsorbed DNA-Cy5 (10 co-localised points in 27 points from Figures 5d and 5b respectively). Single molecules were counted by matching size and intensity of fluorescent points to threshold criteria which separate single molecules from background noise and cosmic rays. Figure 5d shows the level of crosstalk from TMR on the Cy5 channel which is to be 2% as determined by counting only those fluorescent points which fall within the criteria for determining the TMR single molecule fluorescence (2 fluorescence points in 141 from Figures 5c and 5a respectively).

This Example demonstrates that single molecule arrays can be formed, and hybridisation events detected according to the invention. It is expected that the skilled person will realise that modifications may be made to improve the efficiency of the process. For example, improved washing steps, e.g. using a flow cell, would reduce background noise and permit more concentrated solutions to be used, and hybridisation protocols could be adapted by varying the parameters of temperature, buffer, time etc.

## Claims

1. A device comprising an array of molecules capable of interrogation and immobilised on a solid planar surface, each of the molecules being individually resolvable by optical microscopy and detectable as a single molecule fluorescent point and wherein each molecule is immobilised by covalent bonding to the surface, other than at that part of each molecule that can be interrogated, at a density of one molecule per approximately at least 250nm x 250nm.

2. A device comprising an array of molecules capable of interrogation and immobilised on a solid planar surface, each of the molecules being an individually resolvable molecule detectable by optical microscopy as a single fluorescent point, and wherein each molecule is immobilised by covalent bonding to the surface other than that part of the molecule that can be interrogated, and wherein fluorescence from said fluorescent point exhibits single step photobleaching.

3. A device according to claim 1 or 2, wherein the covalent bonding to the surface is without an intermediate microsphere.

4. A device according to claim 2 or 3, wherein each single molecule is separated by a distance of at least 250 nm.

5. A device according to any preceding claim, wherein the molecules are polynucleotides immobilised to the solid support via the 5' terminus, the 3' terminus or via an internal nucleotide.

6. A device according to claim 5, wherein at least one arrayed polynucleotide has a second polynucleotide hybridised thereto.

7. A device according to claim 5 or claim 6, wherein the arrayed polynucleotide comprises a known sequence.

8. A device according to any of claims 1 to 3, wherein the molecules are peptides or proteins.

9. A method for producing a device according to any of claims 1 to 8, comprising dispensing a solution comprising a mixture of molecules onto a solid surface under conditions that permit immobilisation and that minimise aggregation of the molecules in solution.

10. A method for producing a device according to claim 5, comprising
(i) immobilising primer polynucleotides at discrete sites on the surface of a solid support; and
(ii) contacting the immobilised primers with target polynucleotides under hybridising conditions.

11. A method for producing a device according to claim 5, comprising
(i) immobilising first polynucleotides at discrete sites on the surface of a solid support, and hybridising thereto second polynucleotides which form single-stranded overhangs;
(ii) contacting the product of step (i) with target polynucleotides under hybridising conditions;
(iii) ligating the target polynucleotides to the first polynucleotides with a DNA ligase; and, optionally,
(iv) removing the second polynucleotides.

12. A method for forming a spatially addressable array, which comprises determining the sequences of a plurality of polynucleotide molecules immobilised on a device according to any of claims 1 to 8.

13. A method according to claim 12, further comprising the step of hybridising a polynucleotide molecule to its immoblised complement on the array.

14. A method according to claim 12, comprising the repeated steps of: reacting the immobilised polynucleotide with a primer, a polymerase and the different nucleotide triphosphates under conditions sufficient for the polymerase reaction to proceed, wherein each nucleotide triphosphate is conjugated at its 3' position to a label capable of being characterised optically, determining which label (and thus which nucleotide) has undergone the polymerisation reaction, and removing the label.

15. A method for characterising a plurality of first molecules, comprising contacting, under suitable conditions, a spatially addressed array of second molecules with the first molecules, and detecting a binding event, wherein the array is as defined in any of claims 1 to 8.

16. A method according to claim 15, wherein the first molecules comprise a detectable tag.

17. A method according to claim 16, wherein the tag is a fluorophore.

18. A method according to claim 16, wherein the tag is a polynucleotide.

19. A method according to claim 18, wherein the polynucleotide sequence is determined.

20. A method for characterising an organism, comprising the steps of contacting a defined array of polynucleotide molecules immobilised on a solid support with a plurality of fragments of the organism's genomic DNA, under hybridising conditions, and detecting any hybridisation events, to obtain a distinct hybridisation pattern, wherein the array is as defined in claim 5 or claim 6.

21. A method according to claim 20, wherein the organism is human.

22. A method according to claim 20, wherein the organism is bacterial or viral.

23. A method according to any of claims 20 to 22, wherein the fragments of genomic DNA are detectably-labelled.

24. Use of a device according to claim 5, for the capture of a second polynucleotide molecule capable of hybridising with the arrayed polynucleotide, comprising bringing into contact with the device a sample containing the second polynucleotide molecule, under hybridising conditions.

25. Use according to claim 24, wherein the sample is removed from contact with the device, thereby separating from the sample said second polynucleotide hybridised to an arrayed polynucleotide.

26. Use of a device according to any of claims 1 to 8, which comprises identifying each of different molecules on the array, using optical microscopy.

27. Use according to claim 24, wherein the arrayed molecule undergoes repeated interactions with each interaction being monitored.

## Patentansprüche

1. Vorrichtung mit einem Array abrufbarer und auf einer festen, ebenen Fläche immobilisierter Moleküle, wobei jedes der Moleküle einzeln durch optische Mikroskopie auflösbar und als einzelner Molekülfluoreszenzpunkt erfassbar ist, und wobei jedes Molekül, im Gegensatz zu demjenigen Teil jedes Moleküls, das abgerufen werden kann, durch kovalente Bindung an der Fläche mit einem Molekül pro ungefähr mindestens 250 nm x 250 nm immobilisiert ist.

2. Vorrichtung mit einem Array abrufbarer und auf einer festen, ebenen Fläche immobilisierter Moleküle, wobei jedes der Moleküle einzeln durch optische Mikroskopie auflösbar und als einzelner Molekülfluoreszenzpunkt erfassbar ist, und wobei jedes Molekül, im Gegensatz zu demjenigen Teil jedes Moleküls, das abgerufen werden kann, durch kovalente Bindung an der Fläche immobilisiert ist, und wobei die Fluoreszenz aus dem Fluoreszenzpunkt einstufiges Photobleaching zeigt.

3. Vorrichtung nach Anspruch 1 oder 2, wobei die kovalente Bindung an die Fläche ohne eine dazwischen angeordnete Mikrokugel erfolgt.

4. Vorrichtung nach Anspruch 2 oder 3, wobei jedes einzelne Molekül um einen Abstand von mindestens 250 nm getrennt ist.

5. Vorrichtung nach jedem vorhergehenden Anspruch, wobei die Moleküle Polynucleotide sind, die über die 5'-Endstelle, die 3'-Endstelle oder über ein inneres Nucleotid am festen Träger immobilisiert sind.

6. Vorrichtung nach Anspruch 5, wobei mindestens ein im Array angeordnetes Polynucleotid ein daran hybridisiertes zweites Polynucleotid aufweist.

7. Vorrichtung nach Anspruch 5 oder Anspruch 6, wobei das im Array angeordnete Polynucleotid eine bekannte Sequenz umfasst.

8. Vorrichtung nach einem der Ansprüche 1 bis 3, wobei die Moleküle Peptide oder Proteine sind.

9. Verfahren zur Herstellung einer Vorrichtung nach einem der Ansprüche 1 bis 8, das das Abgeben einer ein Molekülgemisch umfassenden Lösung auf eine feste Fläche unter Bedingungen umfasst, die die Immobilisierung zulassen und eine Aggregation der in Lösung befindlichen Moleküle minimieren.

10. Verfahren zur Herstellung einer Vorrichtung nach Anspruch 5, umfassend
(i) Primer-Polynucleotide an einzelnen Stellen auf der Fläche eines festen Trägers zu immobilisieren; und
(ii) die immobilisierten Primer mit Target-Polynucleotiden unter Hybridisierungsbedingungen in Kontakt zu bringen.

11. Verfahren zur Herstellung einer Vorrichtung nach Anspruch 5, umfassend
(i) erste Polynucleotide an einzelnen Stellen auf der Fläche eines festen Trägers zu immobilisieren und zweite Polynucleotide daran zu hybridisieren, die einsträngige Überhänge bilden;
(ii) das Produkt von Schritt (i) unter Hybridisierungsbedingungen mit Target-Polynucleotiden in Kontakt zu bringen;
(iii) die Target-Polynucleotide mit einer DNA-Ligase an die ersten Polynucleotide zu binden, und wahlweise,
(iv) die zweiten Polynucleotide zu entfernen.

12. Verfahren zum Ausbilden eines räumlich adressierbaren Arrays, welches umfasst, die Sequenzen einer Mehrzahl von Polynucleotidmolekülen zu bestimmen, die auf einer Vorrichtung nach einem der Ansprüche 1 bis 8 immobilisiert sind.

13. Verfahren nach Anspruch 12, darüber hinaus den Schritt umfassend, ein Polynucleotidmolekül an sein immobilisiertes Komplement auf dem Array zu hybridisieren.

14. Verfahren nach Anspruch 12, die Wiederholungsschritte umfassend: das immobilisierte Polynucleotid mit einem Primer, einer Polymerase und den unterschiedlichen Nucleotidtriphosphaten unter Bedingungen zur Reaktion zu bringen, die ausreichen, um die Polymerasereaktion vonstatten gehen zu lassen, wobei jedes Nucleotidtriphosphat an seiner 3'-Stelle mit einer Markierung, die optisch gekennzeichnet werden kann, konjugiert wird, zu bestimmen, welche Markierung (und somit welches Nucleotid) die Polymerisierungsreaktion durchgemacht hat, und die Markierung zu entfernen.

15. Verfahren zum Kennzeichnen einer Mehrzahl von ersten Molekülen, das umfasst, ein räumlich adressiertes Array von zweiten Molekülen unter geeigneten Bedingungen mit den ersten Molekülen in Kontakt zu bringen, und ein Bindungsereignis zu erfassen, wobei das Array so wie in einem der Ansprüche 1 bis 8 definiert ist.

16. Verfahren nach Anspruch 15, wobei das erste Molekül eine erfassbare Identifizierungskennzeichnung umfasst.

17. Verfahren nach Anspruch 16, wobei die Identifizierungskennzeichnung ein Fluorophor ist.

18. Verfahren nach Anspruch 16, wobei die Identifizierungskennzeichnung ein Polynucleotid ist.

19. Verfahren nach Anspruch 18, wobei die Polynucleotidsequenz bestimmt wird.

20. Verfahren zum Kennzeichnen eines Organismus, das die Schritte umfasst, ein bestimmtes Array von Polynucleotidmolekülen, die auf einem festen Träger immobilisiert sind, mit einer Mehrzahl von Fragmenten der Gen-DNA des Organismus unter Hybridisierungsbedingungen in Kontakt zu bringen, und alle Hybridisierungsereignisse zu erfassen, um ein eindeutiges Hybridisierungsmuster zu erhalten, wobei das Array wie in Anspruch 5 oder Anspruch 6 definiert ist.

21. Verfahren nach Anspruch 20, wobei der Organismus menschlich ist.

22. Verfahren nach Anspruch 20, wobei der Organismus bakteriell oder viral ist.

23. Verfahren nach einem der Ansprüche 20 bis 22, wobei die Fragmente der Gen-DNA erfassbar markiert sind.

24. Verwendung einer Vorrichtung nach Anspruch 5 zum Einfangen eines zweiten Polynucleotidmoleküls, das zur Hybridisierung mit den in den Arrays angeordneten Polynucleotiden in der Lage ist, umfassend, eine, das zweite Polynucleotidmolekül enthaltende Probe unter Hybridisierungsbedingungen mit der Vorrichtung in Kontakt zu bringen.

25. Verwendung nach Anspruch 24, wobei die Probe außer Kontakt mit der Vorrichtung gebracht wird, wobei das zweite Polynucleotid, das an ein im Array angeordnetes Polynucleotid hybridisiert wurde, von der Probe getrennt wird.

26. Verwendung einer Vorrichtung nach einem der Ansprüche 1 bis 8, die umfasst, jedes von unterschiedlichen Molekülen auf dem Array unter Verwendung optischer Mikroskopie zu identifizieren.

27. Verwendung nach Anspruch 24, wobei das im Array angeordnete Molekül wiederholte Wechselwirkungen durchmacht, wobei jede Wechselwirkung überwacht wird.

## Revendications

1. Dispositif comprenant une rangée de molécules capables d'interrogation et immobilisées sur une surface solide plane, chacune des molécules pouvant être individuellement résolue par microscopie optique et détectée comme un point fluorescent de molécule unique, et dans lequel chaque molécule est immobilisée par liaison covalente à la surface, autre que celle faisant partie de chaque molécule qui peut être interrogée, à une densité d'une molécule pour approximativement au moins 250 nm x 250 nm.

2. Dispositif comprenant une rangée de molécules capables d'interrogation et immobilisées sur une surface solide plane, chacune des molécules étant une molécule pouvant être individuellement résolue et détectée par microscopie optique sous la forme d'un point fluorescent unique, et dans lequel chaque molécule est immobilisée par liaison covalente à la surface autre que celle faisant partie de la molécule qui peut être interrogée, et dans lequel la fluorescence dudit point fluorescent présente un blanchissage optique en une seule étape.

3. Dispositif selon la revendication 1 ou 2, dans lequel la liaison covalente à la surface est réalisée sans microsphère intermédiaire.

4. Dispositif selon la revendication 2 ou 3, dans lequel chaque molécule unique est séparée par une distance d'au moins 250 nm.

5. Dispositif selon l'une quelconque des revendications précédentes, dans lequel les molécules sont des polynucléotides immobilisés sur le support solide via l'extrémité terminale 5', l'extrémité terminale 3' ou via un nucléotide interne.

6. Dispositif selon la revendication 5, dans lequel au moins un polynucléotide en rangées a un second polynucléotide hybridé à celui-ci.

7. Dispositif selon la revendication 5 ou la revendication 6, dans lequel le polynucléotide en rangées comprend une séquence connue.

8. Dispositif selon l'une quelconque des revendications 1 à 3, dans lequel les molécules sont des peptides ou des protéines.

9. Procédé de production d'un dispositif selon l'une quelconque des revendications 1 à 8, comprenant la distribution d'une solution comprenant un mélange de molécules sur une surface solide dans des conditions qui permettent l'immobilisation et qui minimisent l'agrégation des molécules en solution.

10. Procédé de production d'un dispositif selon la revendication 5, comprenant
(i) l'immobilisation des polynucléotides amorces à des sites discrets sur la surface d'un support solide ; et
(ii) la mise en contact des amorces immobilisées avec des polynucléotides cibles dans des conditions d'hybridation.

11. Procédé de production d'un dispositif selon la revendication 5, comprenant
(i) l'immobilisation des premiers polynucléotides à des sites discrets sur la surface d'un support solide, et l'hybridation à ceux-ci de seconds polynucléotides qui forment des débords simple brin ;
(ii) la mise en contact du produit de l'étape (i) avec des polynucléotides cibles dans des conditions d'hybridation ;
(iii) la ligature des polynucléotides cibles aux premiers polynucléotides avec une ADN ligase ; et, facultativement,
(iv) l'élimination des seconds polynucléotides.

12. Procédé de formation d'une rangée spatialement adressable, qui comprend la détermination des séquences d'une pluralité de molecules polynucléotidiques immobilisées sur un dispositif selon l'une quelconque des revendications 1 à 8.

13. Procédé selon la revendication 12, comprenant en outre l'étape consistant à hybrider- une molécule polynucléotidique à son complément immobilisé sur la rangée.

14. Procédé selon la revendication 12, comprenant les étapes répétées consistant à: faire réagir le polynucléotide immobilisé avec une amorce, une polymérase et les différents nucléotides triphosphates dans des conditions suffisantes pour que la réaction de la polymérase ait lieu, dans lequel chaque nucléotide triphosphate est conjugué à sa position 3' à un marqueur pouvant être caractérisé optiquement, déterminer quel marqueur (et ainsi quel nucléotide) a subi la réaction de polymérisation, et éliminer le marqueur.

15. Procédé de caractérisation d'une pluralité de premières molécules, comprenant la mise en contact, dans des conditions adaptées, d'une rangée spatialement adressée de secondes molécules avec les premières molécules, et la détection d'un événement de liaison, dans lequel la rangée est telle que définie dans l'une quelconque des revendications 1 à 8.

16. Procédé selon la revendication 15, dans lequel les premières molécules comprennent un indicateur détectable.

17. Procédé selon la revendication 16, dans lequel l'indicateur est un fluorophore.

18. Procédé selon la revendication 16, dans lequel l'indicateur est un polynucléotide.

19. Procédé selon la revendication 18, dans lequel la séquence polynucléotidique est déterminée.

20. Procédé de caractérisation d'un organisme, comprenant les étapes consistant à mettre en contact une rangée définie de molécules polynucléotidiques immobilisées sur un support solide avec une pluralité de fragments de l'ADN génomique de l'organisme, dans des conditions d'hybridation, et à détecter tout événement d'hybridation, pour obtenir un motif d'hybridation distinct, dans lequel la rangée est telle que définie dans la revendication 5 ou dans la revendication 6.

21. Procédé selon la revendication 20, dans lequel l'organisme est humain.

22. Procédé selon la revendication 20, dans lequel l'organisme est bactérien ou viral.

23. Procédé selon l'une quelconque des revendications 20 à 22, dans lequel les fragments de l'ADN génomique sont marqués de manière détectable.

24. Utilisation d'un dispositif selon la revendication 5, pour la capture d'une seconde molécule polynucléotidique capable de s'hybrider avec le polynucléotide en rangées, comprenant la mise en contact: avec le dispositif d'un échantillon contenant la seconde molécule polynucléotidique, dans des conditions d'hybridation.

25. Utilisation selon la revendication 24, dans laquelle l'échantillon est retiré du contact avec le dispositif, séparant ainsi de l'échantillon ledit second polynucléotide hybridé à un polynucléotide en rangées.

26. Utilisation d'un dispositif selon l'une quelconque des revendications 1 à 8, qui comprend l'identification de chacune des différentes molécules sur la rangée, en utilisant la microscopie optique.

27. Utilisation selon la revendication 24, dans laquelle la molécule en rangées subit des interactions répétées, chaque interaction étant surveillée.
